# EUROPEAN PATENT SPECIFICATION

(11) **EP 1 127 536 B1**
(45) Date of publication and mention of the grant of the patent: **20.04.2005**
(21) Application number: 01103375.0
(22) Date of filing: 13.02.2001
(51) Int. Cl.: A61B 5/00, C12Q 1/68, B01J 19/00

(54) **Biochip**
Biochip
Biopuce

(30) Priority: 22.02.2000 JP 2000044384
(43) Date of publication of application: 29.08.2001
(73) Proprietor: Yokogawa Electric Corporation, Tokyo 180-8750 (JP)
(72) Inventor: Tanaami, Takeo, Tokyo 180-8750 (JP)
(74) Representative: Henkel, Feiler & Hänzel

(56) References cited:
- US-A- 6 013 029

## Description

### Field of the Invention

The present invention relates to a biochip according to the preamble portion of claim 1 for testing such substances as DNA, RNA or protein and, in particular, to a biochip that is extremely safe and can reduce the cost of testing.

### Description of the Prior Art

A biochip, such as a DNA chip, comprises several thousand to several hundred thousand types of known DNA segments, also referred to as DNA probes, deposited in arrays on a substrate. If a solution containing an unknown DNA segment, also referred to as a DNA target, is flowed onto such a DNA chip, DNA segments of the same type combine with each other. This property is utilized so that such a DNA probe wherein such combination has taken place is examined using a biochip reader and thus the sequence of the DNA target, for example, is determined.

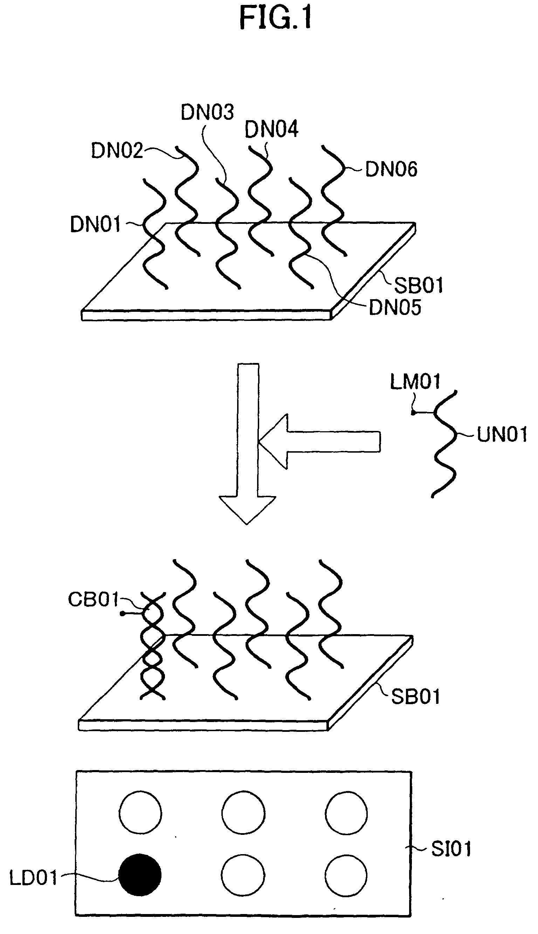

FIG. 1 is a schematic view illustrating an example of hybridization seen in such a biochip as discussed above. In FIG. 1, six DNA probes indicated by DN01, DN02, DN03, DN04, DN05 and DN06 are deposited in arrays on a substrate SB01, thus composing a DNA chip.

The symbol UN01 in FIG. 1 indicates a DNA target, which is previously marked with a fluorescent marker as indicated by LM01. When hybridized to the DNA chip, this DNA target combines with a DNA probe whose sequence is complementary.

For example, the DNA target UN01 combines with the DNA probe DN01, as indicated by CB01 in FIG. 1.

Using a biochip reader, excitation light is irradiated at the DNA chip thus hybridized, in order to detect fluorescent light produced at the fluorescent marker. Consequently, it is possible to know which of the DNA probes the DNA target has combined with.

For example, in an image resulting from scanning a DNA chip indicated by SI01 in FIG. 1, fluorescent light is observed only at a spot where the DNA combination CB01 has been produced. This means fluorescent light is detected only from the spot indicated by LD01 in FIG. 1.

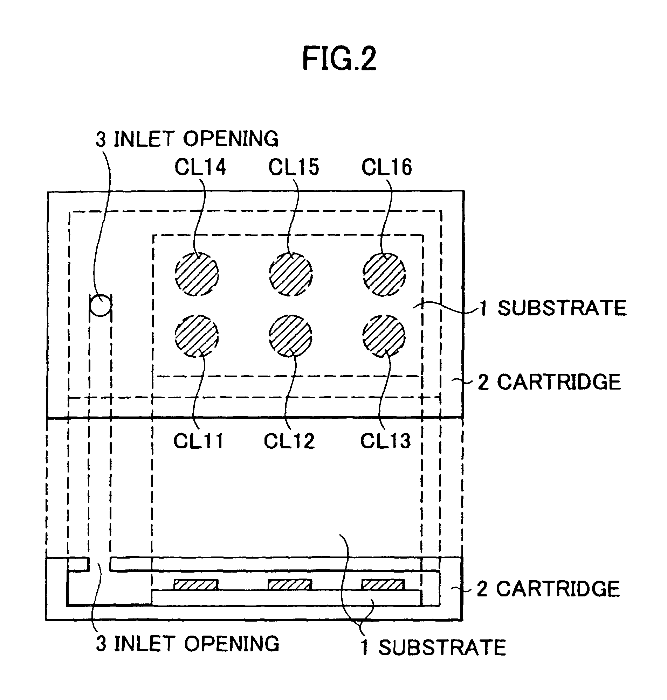

FIG. 2 shows the schematic top and cross-sectional views of such a prior art biochip as described above. In the following explanation, a DNA chip is cited as the biochip. The biochip comprises a substrate 1 on which known DNA segments are deposited in arrays (hereinafter simply referred to as "substrate 1") as DNA probes, a cartridge 2 wherein the substrate 1 is housed and to which a solution containing a DNA target previously marked with a fluorescent marker is introduced, and an inlet opening 3 formed on the cartridge 2 through which to introduce the solution.

The cartridge 2 is made of a material permeable to both excitation light and fluorescent light produced thereby at the marker. Cells CL11, CL12, CL13, CL14, CL15 and CL16, in each of which a plurality of DNA probes of the same type are placed, are deposited in arrays on the substrate 1.

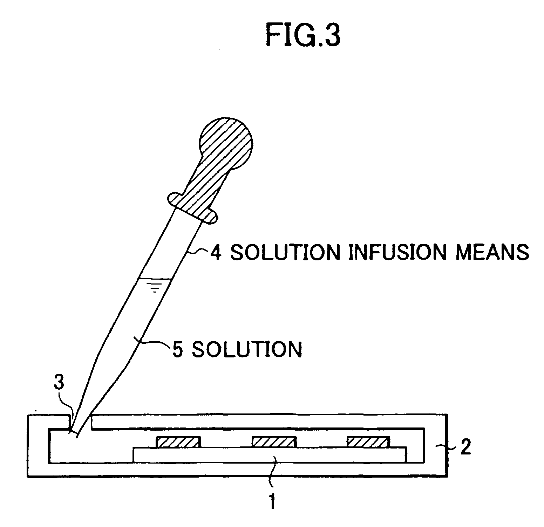

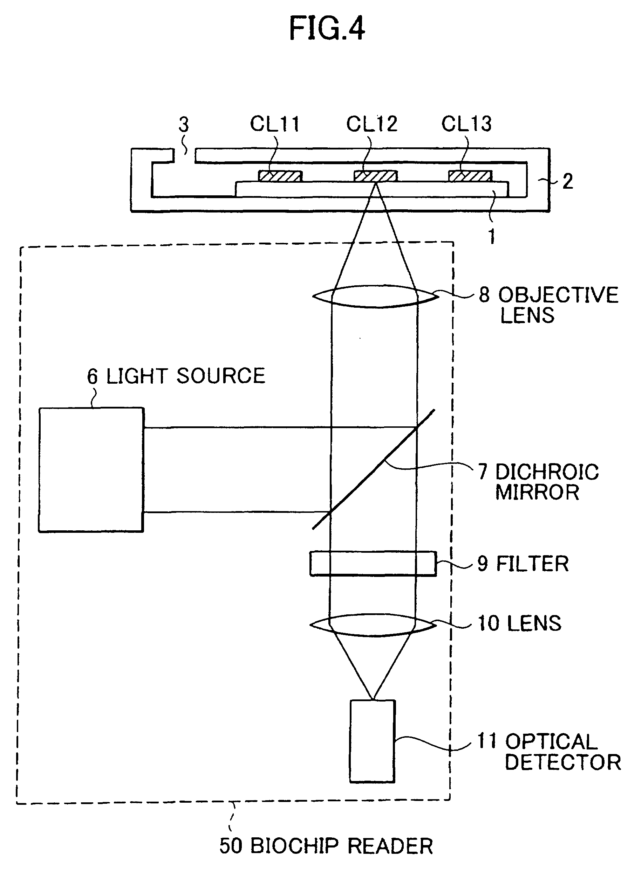

Now the method of testing DNA or other substances using the biochip shown in FIG. 2 is explained with reference to FIGS. 3 and 4. FIG. 3 is a schematic view illustrating an example of introducing the solution into the cartridge 2, whereas FIG. 4 is a schematic view illustrating an example of scanning a hybridized DNA chip using a biochip reader to determine the sequence of target DNA, for example.

In a first step, blood is collected using a syringe from a person to be tested, such as a patient. A solution that has undergone preprocessing is then introduced through an inlet opening 3 into the cartridge 2.

In FIG. 3, components indicated by the numerals 1 to 3 are the same as those shown in FIG. 2, and solution infusion means 4, such as a pipette, is loaded with a preprocessed solution 5. As shown in FIG. 3, the tip of the solution infusion means 4 is inserted in the inlet opening 3 and the solution 5 inside the solution infusion means 4 is injected into the cartridge 2.

Note that the above-described preprocessing refers to a series of processes wherein lymphocytes are separated from the collected blood, then DNA is extracted from the separated lymphocytes, and finally the extracted DNA is marked with a fluorescent marker.

In a second step, the substrate 1 is soaked with the solution introduced into the cartridge 2 to allow a DNA target in the solution to hybridize with DNA probes placed in each cell on the substrate 1.

In a final step, the hybridized substrate 1 is scanned using such a biochip reader as a biochip reader 50 shown in FIG. 4 so that, for example, the sequence of the DNA target is determined.

In FIG. 4, the components 1 to 3 and the cells CL11, CL12 and CL13 are referenced in the same way as those of FIG. 3.

Light emitted from a light source 6, such as a laser light source, is reflected by a dichroic mirror 7 as excitation light and focused through an objective lens 8 onto cells on the substrate 1. For example, the excitation light is focused onto the cell CL12 in FIG. 4.

Fluorescent light produced at the cell CL12 on the substrate 1 becomes parallel light as it travels through the objective lens 8, and is projected onto the dichroic mirror 7. The fluorescent light thus projected transmits through the dichroic mirror 7. The fluorescent light thus transmitted travels through a filter 9 and is condensed onto an optical detector 11 by a lens 10.

The spots whereupon the excitation light is focused are scanned by a drive means which is not shown in FIG. 4. For example, the cartridge 2 or biochip reader 50 itself is scanned so that the excitation light is irradiated at the remaining cells CL11 and CL13 on the substrate 1.

Consequently, it is possible to determine the sequence of an introduced DNA target by identifying the position of a cell on the substrate 1 where fluorescence has taken place.

Recently, however, such test samples as blood are often found to be contaminated with a virus such as HIV. Therefore, there is a growing tendency that for safety reasons, such medical appliances as syringes are not recycled by cleaning or sterilization; rather, disposable medical equipment is used instead. In contrast, the method of introducing a solution shown in FIG. 3 involves the risk of the operator becoming infected with a virus, such as HIV, as a result of accidental contact with the solution due to mishandling. This risk exists because the method always involves transferring the solution from the solution infusion means 4 or the like to the cartridge 2.

Another problem with the prior art method is that the cost of testing increases since more than one kind of medical equipment must be disposed off, including syringes, appliance used for preprocessing purposes, solution infusion means, DNA chips, and so on.

US-A-6 013 029 discloses a device for monitoring the concentration of a substance or a group of substances in a body fluid of a living human or animal body. The device comprises an interface, a detector, a flow channel or conduit connecting the interface to the detector, and means for maintaining a flow along the interface and the detector. Between the interface and the detector the device is provided with the selector and a preoxidator upstream from the selector. The interface is for example provided in the form of a microdialysis tube which is to be inserted subcutaneously. The detector is provided in the form of an amperometric electrochemical detector and the preoxidator is essentially identical to an electrochemical detector but preferably has a relatively large oxidation surface and a potential which is set at a higher level than the potential of the electrochemical detector. In the selector, a glucoseoxidase enzyme is physically immobilized between two cellulose nitrate membranes. The whole device can be for example incorporated in a catheter.

### SUMMARY OF THE INVENTION

An object of the present invention is to provide a biochip that is extremely safe and can reduce the cost of testing.

According to the invention there is provided a biochip as defined in claim 1. Preferred embodiments are defined in the dependent claims.

### BRIEF DESCRIPTION OF THE DRAWINGS

FIG. 1 is a schematic view illustrating an example of hybridization seen in a biochip.
FIG. 2 shows the schematic top and cross-sectional views of a prior art biochip.
FIG. 3 is a schematic view illustrating an example of introducing a solution into a cartridge.
FIG. 4 is a schematic view illustrating an example of scanning a hybridized DNA chip using a biochip reader to determine the sequence of target DNA, for example.
FIG. 5 is a cross-sectional view of one embodiment of a biochip according to the present invention.
FIG. 6 is a schematic view illustrating a way the embodiment according to the present invention is applied.

### DETAILED DESCRIPTION OF THE PREFERRED EMBODIMENTS

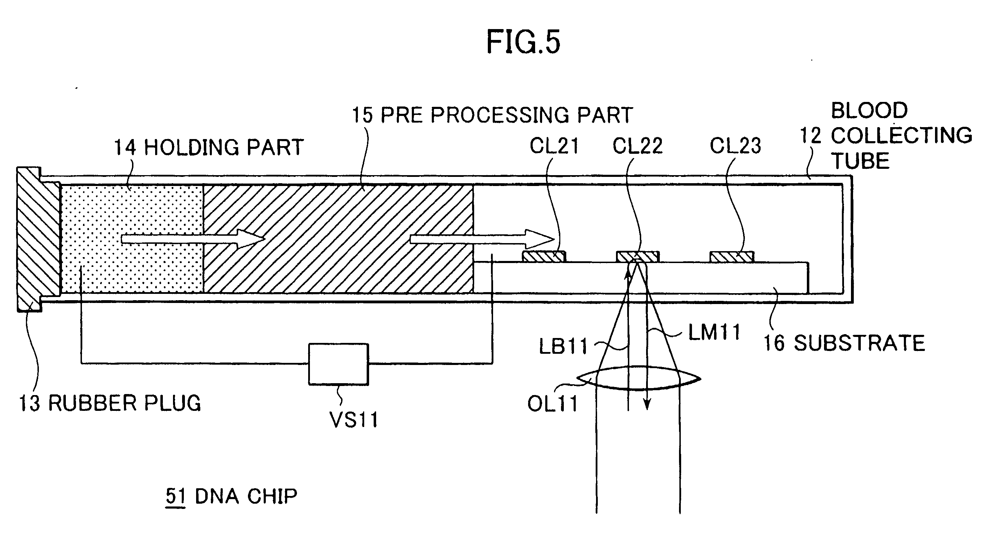

The present invention will now be described in detail with reference to the accompanying drawings. FIG. 5 is a cross-sectional view of one embodiment of a biochip according to the present invention.

A DNA chip (biochip) 51 comprises a blood collecting tube 12, instead of a conventional spit tube, which is inserted in a syringe cylinder in order to collect blood and is permeable to excitation light and fluorescent light produced thereby at a fluorescent marker. A substrate 16 on which DNA probes are deposited in arrays as samples (hereinafter simply referred to as the substrate 16) is located in the innermost section of the blood collecting tube 12. Additionally, a preprocessing block 15, wherein the preprocessing described earlier is carried out, is located in the intermediate section of the blood collecting tube 12. Space in the outermost section of the blood collecting tube 12 serves as a collection block 14 for temporarily storing collected blood. The innermost section of the blood collecting tube 12 is kept under negative pressure against the collection block 14, or is kept under vacuum for example, and a rubber plug 13 whose middle area has a thin wall through which a needle can pierce, is placed onto the opening of the blood collecting tube 12 in order to make the tube airtight.

Cells CL21, CL22 and CL23, in each of which a plurality of DNA probes of the same type are deposited, are deposited in arrays on the substrate 16.

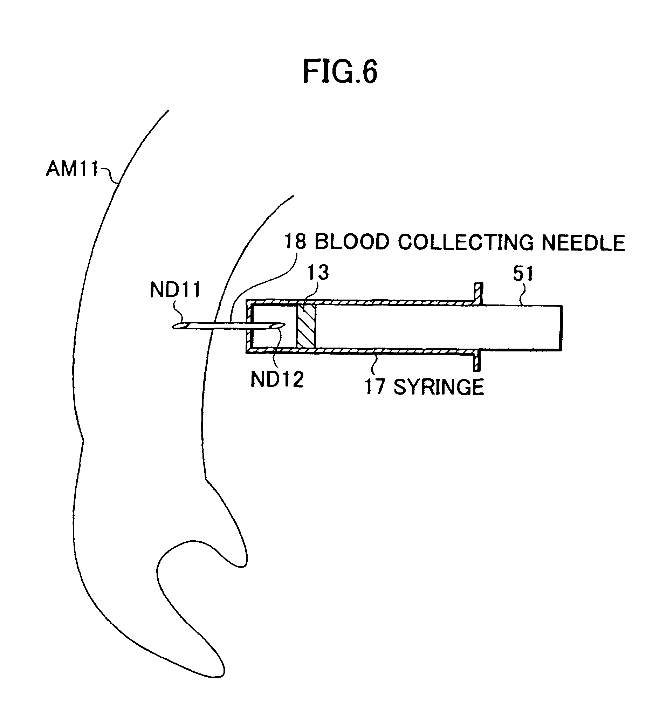

Now the way the embodiment of FIG. 5 is applied and operated is described by referring to FIG. 6 which is a schematic view illustrating the application and operation. In FIG. 6, components indicated by 13 and 51 are referenced in the same way as those of FIG. 5.

A blood collecting needle 18 is located on the side opposite to the opening of a syringe 17 and has pierceable ends on both sides thereof, as indicated by ND11 and ND12 in FIG. 6. Instead of a conventional spit tube, a DNA chip 51 is inserted through the opening of the syringe 17. At this point, one end of the blood collecting needle 18 indicated by ND12 in FIG. 6 pierces through the middle area of a rubber plug 13 on the DNA chip 51 so that the end of the needle is connected to the DNA chip 51.

Under the above-described condition, the arm of the person to be tested, such as a patient, indicated by AM11 in FIG. 6 is pricked with the other end of the blood collecting needle 18 indicated by ND11. Consequently, blood is collected through the blood collecting needle 18 into the collection block 14 (not shown) of the DNA chip 51, whereby blood collection is accomplished.

After the blood collection, the blood collecting needle 18 is removed from the arm of the person to be tested and the DNA chip 51 is removed from the syringe 17. At this point, a pinhole in the part of the rubber plug 13 where the needle was pierced automatically closes due to the elasticity of the rubber plug 13, whereby the blood collecting tube 12 is kept airtight.

The blood thus collected into the collection block 14 then infiltrates toward the innermost section of the blood collecting tube 12 under negative pressure against the collection block 14. Thus, the blood is introduced to the preprocessing block 15. In the preprocessing block 15, a series of processes is carried out, i.e., lymphocytes are separated from the collected blood, DNA is extracted from the separated lymphocytes, and the extracted DNA is marked with a fluorescent marker, as described earlier.

The preprocessed DNA infiltrates into the innermost section of the blood collecting tube 12 under negative pressure against the collection block 14. The collected blood is thus introduced into the section where the substrate 16 is located. Then, as described earlier, a DNA target marked with a fluorescent marker is hybridized with DNA probes so that DNA segments whose sequences are complementary combine with each other.

Under the above-described condition, excitation light is irradiated at the substrate 16 thus hybridized, using the biochip reader discussed earlier, and fluorescent light produced at the fluorescent marker is detected. Consequently, it is possible to know which of the DNA probes the DNA target has combined with.

For example, the excitation light of a biochip reader (not illustrated) indicated by LB11 in FIG. 5 is focused with an objective lens indicated by OL11 onto a cell on the substrate 16 indicated by CL22. Then, by detecting fluorescent light produced at the cell CL22 as indicated by LM11, it is possible to identify the DNA target.

Then, upon completion of testing all that remains to be done is simply to dispose of the syringe 17 and DNA chip 51 used for blood collection as medical waste. This approach eliminates the need for disposing of any additional medical equipment, including appliances used for preprocessing purposes and solution infusion means, as seen in the prior art method, whereby the cost of testing is reduced.

The biochip of the present invention also eliminates the need for an examiner to transfer collected blood to a DNA chip. Consequently, the examiner avoids the risk of being infected with a virus such as HIV as a result of accidental contact with the collected blood due to mishandling, whereby safety is ensured.

Accordingly, the biochip of the present invention comprising the blood collecting tube 12, which includes the collection block 14, the preprocessing block 15, and the substrate 16 on which probes are deposited in arrays, is extremely safe and can reduce the cost of testing.

Although a DNA chip is cited as the biochip in the explanation of FIG. 5 or other drawings, the present invention is by no means limited to DNA chips. The biochip may be of such type that RNA, protein or sugar chain samples are deposited in arrays on a substrate.

In this case, the RNA samples undergo hybridization as with DNA samples, whereas the protein samples are submitted to an antigen-antibody reaction. In either case, a probe combines with a target.

Also in an earlier explanation, the block wherein the substrate 16 is located is kept under negative pressure against the collection block 14 as a method of introducing such a test sample as blood from the collection block 14 to the preprocessing block 15. The present invention is not limited to this method, however.

One alternative method may be forming electrodes on both ends of the preprocessing block 15 and applying a voltage externally across them, so that blood or the like is introduced by means of electrophoresis. For example, DNA is introduced in the direction from the preprocessing block 15 to the substrate 16 when the polarities of a voltage source indicated by VS11 in FIG. 5 are configured so that the substrate 16 side of the voltage source is positive and the collection block 14 side thereof is negative. This is because the DNA is negatively charged in a case where the biochip is a DNA chip.

Another alternative method may be simply using natural diffusion based on osmotic pressure to introduce blood or the like. Yet another alternative method may be to provide the innermost section of the blood collecting tube 12 with an evacuation port, thereby evacuating the innermost section externally and thus introducing blood or the like by means of osmosis.

Although fluorescent light detection using a fluorescent markers is applied in the embodiment of FIG. 5, such an alternative method as detecting electric current changes corresponding with hybridization or conducting mass analysis may be used instead.

In the method based on electric current changes, for example, a molecule known as an intercurrenter is slipped into a double chain after hybridization. Then, an electric current is measured since it only flows through those electrodes where hybridization has taken place. In the case of mass analysis, ionized DNA molecules picked from each cell are made to fly in a vacuum and their molecular weight is determined from the difference in time each DNA molecule arrives at a given electrode.

As is evident from the above description, the present invention has the following advantageous effects:

According to aspects of the present invention as described in claims 1 to 5, a collection block, a preprocessing block, and a substrate on which probes are deposited in arrays are included in a blood collecting tube, thus eliminating the need for an examiner to transfer collected blood to a biochip. Consequently, the examiner avoids the risk of being infected with a virus such as HIV as a result of accidental contact with the collected blood due to mishandling, whereby safety is ensured.

Another advantage is that upon completion of testing a biochip, all that remains to be done is simply to dispose of the syringe used for blood collection and the biochip as medical waste. This approach eliminates the need for disposing of any additional medical equipment, including appliances used for preprocessing purposes and solution infusion means, as seen in the prior art method, whereby the cost of testing is reduced.

According to an aspect of the present invention as described in claim 6, a preprocessing block derives DNA from blood collected into a collection block. Then, since samples deposited in arrays on a substrate are also samples of DNA, a DNA probe and a DNA target whose sequences are complementary combine with each other as a result of hybridization. Consequently, it is possible to determine the sequence of the DNA target.

According to an aspect of the present invention as described in claim 7, a preprocessing block derives RNA from blood collected into a collection block. Then, since samples deposited in arrays on a substrate are also samples of RNA, an RNA probe and an RNA target whose sequences are complementary combine with each other as a result of hybridization. Consequently, it is possible to determine the sequence of the RNA target.

According to an aspect of the present invention as described in claim 8, a preprocessing block derives protein from blood collected into a collection block. Then, since samples deposited in arrays on a substrate are also samples of protein, a protein probe and a protein target whose sequences are complementary combine with each other as a result of antigen-antibody reaction. Consequently, it is possible to determine the sequence of the protein target.

## Claims

1. A biochip (51) comprising a blood collecting tube (12) containing, in sequence from an opening of said blood collecting tube (12):
a collection block (14) for retaining collected blood,
a preprocessing block (15) for deriving a target from said collected blood, and
a substrate (16) on which probes are deposited in arrays,
**characterized by** further comprising
means for introducing the collected blood from said collection block (14) to said preprocessing block (15), and
a rubber plug (13) airtightly closing said opening of the collection tube (12).

2. The biochip of claim 1, wherein said means for introducing the collected blood from said collection block (14) to said preprocessing block (15) comprises natural diffusion.

3. The biochip of claim 1, wherein said means for introducing the collected blood from said collection block (14) to said preprocessing block (15) comprises a negative pressure kept in a section where said substrate (16) is located against said collection block (14) so that blood collected into said collection block (14) can be introduced to said preprocessing block (15) by means of osmosis based on said negative pressure.

4. The biochip of claim 1, wherein said means for introducing the collected blood from said collection block (14) to said preprocessing block (15) comprises an evacuation port at a section where said substrate (16) is located so that said section can be subjected to evacuation and thereby depressurization so that blood collected into said collection block (14) can be introduced to said preprocessing block (15) by means of osmosis based on said evacuation.

5. The biochip of claim 1, wherein said means for introducing the collected blood from said collection block (14) to said preprocessing block (15) comprises electrodes on both ends of said preprocessing block (15) so that a voltage can be applied externally so that blood collected into said collection block (14) can be introduced to said preprocessing block (15) by means of electrophoresis.

6. The biochip of any one of claims 1 to 5, wherein said preprocessing block (15) is adapted to derive DNA from blood collected into said collection block (14).

7. The biochip of any one of claims 1 to 5, wherein said preprocessing block (15) is adapted to derive RNA from blood collected into said collection block (14).

8. The biochip of any one of claims 1 to 5, wherein said preprocessing block (15) is adapted to derive protein from blood collected into said collection block (14).

## Patentansprüche

1. Biochip (51) mit einer Blutsammelröhre (12), die der Reihe nach von einer Öffnung der Blutsammelröhre (12) enthält:
einen Sammelblock (14) zum Zurückhalten gesammelten Blutes,
einen Vorbehandlungsblock (15) zum Ableiten eines Targets von dem gesammelten Blut, und
ein Substrat (16), auf dem Sonden in Arrays angeordnet sind,
**dadurch gekennzeichnet, dass** er ferner umfasst
Mittel zum Einbringen des gesammelten Blutes von dem Sammelblock (14) zu dem Vorbehandlungsblock (15), und
einen Gummistopfen (13), der die Öffnung der Sammelröhre (12) luftdicht verschließt.

2. Biochip nach Anspruch 1, wobei das Mittel zum Einbringen des gesammelten Blutes von dem Sammelblock (14) zu dem Vorbehandlungsblock (15) natürliche Diffusion umfasst.

3. Biochip nach Anspruch 1, wobei das Mittel zum Einbringen des gesammelten Blutes von dem Sammelblock (14) zu dem Vorbehandlungsblock (15) einen Unterdruck umfasst, der in einem Abschnitt gehalten wird, in dem das Substrat (16) gegen den Sammelblock (14) so angeordnet ist, dass in den Sammelblock (14) aufgenommenes Blut mittels Osmose, basierend auf dem Unterdruck, zu dem Vorbehandlungsblock (15) eingebracht werden kann.

4. Biochip nach Anspruch 1, wobei das Mittel zum Einbringen des gesammelten Blutes von dem Sammelblock (14) zu dem Vorbehandlungsblock (15) einen Evakuierungsanschluß an einem Abschnitt umfasst, an dem das Substrat (16) positioniert ist, dass der Abschnitt einer Evakuierung und dadurch einer Druckminderung unterzogen werden kann, so dass in den Sammelblock (14) aufgenommenes Blut mittels Osmose basierend auf der Evakuierung in den Vorbehandlungsblock (15) eingebracht werden kann.

5. Biochip nach Anspruch 1, wobei das Mittel zum Einbringen des gesammelten Blutes von dem Sammelblock (14) zu dem Vorbehandlungsblock (15) Elektroden an beiden Enden des Vorbehandlungsblocks (15) umfasst, so dass eine Spannung extern so angelegt werden kann, dass in den Sammelblock (14) aufgenommenes Blut mittels Elektrophorese zu dem Vorbehandlungsblock (15) eingebracht werden kann.

6. Biochip nach einem der Ansprüche 1 bis 5, wobei der Vorbehandlungsblock (15) aus dem in den Sammelblock (14) aufgenommenen Blut DNA ableiten kann.

7. Biochip nach einem der Ansprüche 1 bis 5, wobei der Vorbehandlungsblock (15) aus dem in den Sammelblock (14) aufgenommenen Blut RNA ableiten kann.

8. Biochip nach einem der Ansprüche 1 bis 5, wobei der Vorbehandlungsblock (15) aus dem in den Sammelblock (14) aufgenommenen Blut Protein ableiten kann.

## Revendications

1. Biopuce (51) comprenant un tube (12) collecteur de sang contenant, en séquence à partir d'une ouverture du tube (12) collecteur de sang :
un bloc (14) collecteur pour retenir du sang collecté,
un bloc (15) de prétraitement pour déduire une cible à partir du sang collecté, et
un substrat (16) sur lequel des sondes sont déposées suivant des réseaux,
**caractérisée en ce qu'**elle comprend en outre
des moyens d'introduction du sang collecté du bloc (14) collecteur au bloc (15) de prétraitement, et
un bouchon (13) en caoutchouc fermant d'une manière étanche à l'air l'ouverture du tube (12) collecteur.

2. Biopuce suivant la revendication 1, dans laquelle les moyens d'introduction du sang collecté du bloc (14) collecteur au bloc (15) de prétraitement comprennent une diffusion naturelle.

3. Biopuce suivant la revendication 1, dans laquelle les moyens d'introduction du sang collecté du bloc (14) collecteur au bloc (15) de prétraitement comprennent une dépression maintenue dans une section dans laquelle le substrat (16) est placé contre le bloc (14) collecteur de sorte que du sang collecté dans le bloc (14) collecteur peut être introduit dans le bloc (15) de prétraitement au moyen d'une osmose basée sur la dépression.

4. Biopuce suivant la revendication 1, dans laquelle les moyens d'introduction du sang collecté du bloc (14) collecteur au bloc (15) de prétraitement comprennent un orifice de mise sous vide à une section où le substrat (16) est placé de façon à ce que cette section puisse être soumise à un vide et ainsi à une dépression de sorte que du sang collecté dans le bloc (14) collecteur peut être introduit dans le bloc (15) de prétraitement au moyen d'une osmose basée sur cette mise sous vide.

5. Biopuce suivant la revendication 1, dans laquelle les moyens d'introduction du sang collecté du bloc (14) collecteur au bloc (15) de prétraitement comprennent des électrodes aux deux extrémités du bloc (15) de prétraitement de sorte qu'une tension peut être appliquée de l'extérieur de manière à ce que du sang collecté dans le bloc (14) collecteur puisse être introduit dans le bloc (15) de prétraitement au moyen d'une électrophorèse.

6. Biopuce suivant l'une quelconque des revendications 1 à 5, dans laquelle le bloc (15) de prétraitement est apte à déduire l'ADN du sang collecté dans le bloc (14) collecteur.

7. Biopuce suivant l'une quelconque des revendications 1 à 5, dans laquelle le bloc (15) de prétraitement est apte à déduire de l'ARN du sang collecté dans le bloc (14) collecteur.

8. Biopuce suivant l'une quelconque des revendications 1 à 5, dans laquelle le bloc (15) de prétraitement est apte à déduire une protéine du sang collecté dans le bloc (14) collecteur.
